# EUROPEAN PATENT SPECIFICATION

(11) **EP 2 629 768 B1**
(45) Date of publication and mention of the grant of the patent: **20.08.2014**
(21) Application number: 11785099.0
(22) Date of filing: 13.10.2011
(51) Int. Cl.: A61K 31/185, A61P 29/02

(54) **SODIUM 2 -MERCAPTOETHANE SULFONATE FOR USE IN THE TREATMENT OF LUMBAR PAIN**
NATRIUM-2-MERCAPTOETHAN-SULFONAT ZUR VERWENDUNG BEI DER BEHANDLUNG VON LENDENSCHMERZEN
2-MERCAPTOÉTHANE SULFONATE DE SODIUM POUR L'UTILISATION DANS LE TRAITEMENT D'UNE DOULEUR LOMBAIRE

(30) Priority: 18.10.2010 IT RM20100554
(43) Date of publication of application: 28.08.2013
(73) Proprietor: Universita' Campus Bio-Medico di Roma, 00128 Rome (IT)
(72) Inventor: CARASSITI, Massimiliano, I-00128 Rome (IT); AGRO', Felice Eugenio, I-00136 Rome (IT); DENARO, Vincenzo, I-00128 Rome (IT); DI MARTINO, Alberto Corrado, I-00128 Rome (IT)
(74) Representative: Germinario, Claudio
(86) International application number: PCT/IB2011/054529
(87) International publication number: WO 2012/052888

(56) References cited:
- BORG P A J ET AL: "Hyaluronidase in the management of pain due to post-laminectomy scar tissue", PAIN, ELSEVIER SCIENCE PUBLISHERS, AMSTERDAM, NL, vol. 58, no. 2, 1 August 1994 (1994-08-01) , pages 273-276, XP024379905, ISSN: 0304-3959, DOI: DOI:10.1016/0304-3959(94)90209-7 [retrieved on 1994-08-01]
- DENARO VINCENZO ET AL: "Effectiveness of a mucolythic agent as a local adjuvant in revision lumbar spine surgery.", EUROPEAN SPINE JOURNAL : OFFICIAL PUBLICATION OF THE EUROPEAN SPINE SOCIETY, THE EUROPEAN SPINAL DEFORMITY SOCIETY, AND THE EUROPEAN SECTION OF THE CERVICAL SPINE RESEARCH SOCIETY DEC 2008 LNKD- PUBMED:18839224, vol. 17, no. 12, December 2008 (2008-12), pages 1752-1756, XP019659721, ISSN: 1432-0932 cited in the application
- DENARO V ET AL: "Effect of a mucolytic agent on collagen fibres: An optical and polarized light histological study", EUROPEAN JOURNAL OF ORTHOPAEDIC SURGERY AND TRAUMATOLOGY 2001 FR LNKD- DOI:10.1007/BF01686890, vol. 11, no. 4, 2001, pages 209-212, XP002629171, ISSN: 0948-4817

## Description

The present invention refers to sodium 2-mercaptoethane sulfonate, pharmaceutically acceptable salts thereof and pharmaceutical compositions comprising it for epidural use in the treatment of lumbar pain.

### STATE OF THE PRIOR ART

Lumbar pain represents one of the most common chronic problems having a remarkable impact in economic and social terms and above all on individual well-being. The reference treatment for lumbar pain is currently represented by infiltration of steroids via the epidural route. Response to epidural steroid injection is best when the pain is of recent onset, acute or chronic relapsed. This treatment, when administered for a long time, can cause various side effects harmful to the patient.

Alternative treatments that can be proposed to the patient with lumbar pain are physical therapy, hydrotherapy, analgesics administration and percutaneous electrical neurostimulation.

In many cases none of these treatments is conclusive, and the patient is forced to undergo surgical treatment of the lumbar rachis. Surgical treatment may cause Failed Back Surgery Syndrome (FBSS), a wording that denotes some possible complications, or better, side effects, linked to disc herniation decompression surgery.

FBSS is a pathology often depending on the onset of a fibrosis with dense cicatricial tissue in the peridural region, developing in the wake of the post-surgical hematoma that originates at the level of the lamines and the deep surface of paravertebral muscles. The fibrosis tends to extend at the level of the spinal canal and strongly adheres to the dura mater and the nerve roots, causing ischemic-type lesions and adherences: peridural and periradicular fibrosis represents one of the foremost determinants of FBSS.

FBSS diagnosis implies three key concepts: (1) the patient underwent surgery on the lumbar rachis and did not exhibit an improvement, or developed a worsening of the clinical picture; (2) the patient exhibits a pain refractory to conservative treatment, and can again be referred to surgical treatment (revision surgery) in order to alleviate the symptomatology; (3) often, however, following the new surgical intervention a persistence/ingravescence of pain symptomatology is highlighted.

The occurrence by far most frequent is represented by cicatritial outcomes: as is visible at skin level in the incision site, even where disc material has been removed cicatritial tissue is formed, as an outcome of the surgical act. Such tissue can give no sign of its presence for a variable time, and the patient rightly believes to be healed. Sometimes, even years after surgery, by effect of modifications that onset on the spine this anelastic tissue, which has "frozen" an array of structures, not being able to follow the modifications of the spine, begins to exert a mechanical-type action on the root; the patient again begins to feel the same symptoms and disorders for which he/she had been operated, and is forced to start over, with scarce chances of success, the therapeutic treatments followed prior to surgery.

Repeated injection of hyaluronidase through the intervertebral foramen into the scar tissue resulted in periods of appreciably reduced pain in a patient with rebound radicular syndrome due to post-laminectomy scarring (Borg et al.).

Therefore, in light of the drawbacks mentioned above, the need to propose novel treatments for the treatment of lumbar pain is highly felt.

### SUMMARY OF THE INVENTION

### Sodium 2-mercaptoethane sulfonate (MESNA or Uromitexan) having formula (I)

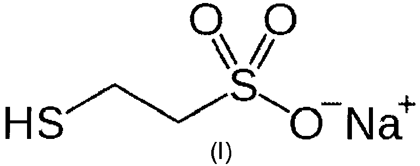

Is a thiolic compound carrying out a protective action towards urotoxic (adverse) events (inflammatory-hemorrhagic processes of the bladder mucosa) induced by oxazaphosphorinic antiblastic agents: it has a marked organotropism towards the urinary tract, and, by two types of chemical reactions, inactivates acrolein (a highly urotoxic oxazaphosphorin demolition product) and 4-hydroxy metabolites.

The lytic action of sodium 2-mercaptoethane sulfonate on collagen fibers, and a topical application of this compound in the chemical dissection of peridural fibrosis during surgical procedures for lumbar spine revision are known in the literature (Denaro et al. Eur Spine J. 2008).

The Inventors have surprisingly discovered that administration of sodium 2-mercaptoethane sulfonate via the epidural route causes a strong reduction of lumbar pain, e.g. in subjects in which lumbar pain is associated to Failed Back Surgery Syndrome.

The epidural administration mode, preferably with injection technique by "low-resistance" and "liquid mandrel" syringe, has innovative and minimally invasive features with respect to intraoperative instillation on the spine, as reported in Denaro et al.

The percutaneous epidural administration route results in the compound exerting its action *in situ,* and in the lytic action on the fibrous bands, fostering a significant reduction in the painful symptomatology.

Therefore, the invention offers the option of treating patients with lumbar pain, having e.g. a case history of postoperative chronic low back pain, in a minimally invasive way with respect to a further surgical operation, to percutaneous electrical neurostimulation, to pharmacological treatments such as steroid infiltration via the epidural route, to the taking of analgesics, comprising high-dosage opioids, via the transcutaneous, oral, intravenous and intrathecal route.

The non-toxicity and the low cost of the compound represent a further advantage in terms of containment of health care expenses.

A first object of the present invention is sodium 2-mercaptoethane sulfonate for epidural use in the treatment of lumbar pain.

A second object of the present invention is a pharmaceutical composition comprising sodium 2-mercaptoethane sulfonate and one or more carriers and/or diluents and/or excipients for epidural use in the treatment of lumbar pain.

Still further advantages, as well as the features and the modes of use of the present invention will be made evident in the following detailed description of some preferred embodiments thereof, given by way of example and not for limitative purposes.

### DETAILED DESCRIPTION OF THE FIGURES

Figure 1: flow chart as an exemplary diagram of a study under way for the treatment of FBSS.
Figure 2: peridural infiltration procedure with injection technique by "low-resistance" and "liquid mandrel" syringe.
Figure 3: low back pain variation (quantified by NRS visuo-analogic scales) post-procedure of peridural injection of sodium 2-mercaptoethane sulfonate in the three positions: lying, sitting and standing position.
Figure 4: Oswestry Disability Index variation post-procedure of peridural injection of sodium 2-mercaptoethane sulfonate.

### DETAILED DESCRIPTION OF THE INVENTION

The present invention refers to sodium 2-mercaptoethane sulfonate for epidural use in the treatment of lumbar pain.

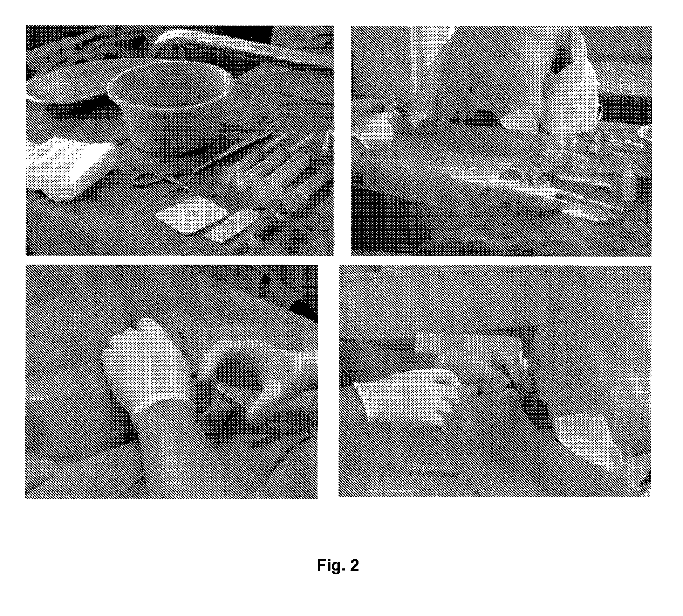

Administration of sodium 2-mercaptoethane sulfonate via the epidural route of administration could be carried out by the methods and means known to an expert in the art, e.g. by single peridural infiltration with liquid mandrel (LORS) technique and suitable "low-resistance" syringe connected to the Tuohy needle (e.g., Figure 2).

Epidural use of sodium 2-mercaptoethane sulfonate is intended for the treatment of lumbar pain in any form, e.g. chronic or acute, and associated to any pathology, like, e.g., lumbosciatic pain or crural pain due to spinal disc herniation, tumoral lesions that compress nerves, metabolic or post-radiotherapy radiculopathies.

Preferably, sodium 2-mercaptoethane sulfonate via the epidural route could be used in the therapeutic treatment of failed back surgery syndrome (FBSS). The category of patients suffering from FBSS includes patients that, following surgery for spinal disc, lumbar canal stenosis, degenerative and isthmic spondylolisthesis, did not exhibit an improvement from a clinical standpoint but an exacerbation of painful symptomatology. Pain features are variable, in fact pain may be prominently localized at the axial level (low back), or it may be associated also to a typically radicular involvement (according to a metameric distribution). With regard to FBSS classification, a parameter such as that of time elapsed between surgery and onset of symptoms may be used. The wording "immediate onset" is adopted when pain is immediately present following the intervention or onsets within 2-3 weeks: the causes of this typology of pain are referable to non-removal of disc fragments, incomplete decompression, and erroneous identification of the zone to be operated (level error). Onset is defined as "mid-term" when symptomatology appears between 4 weeks and 6 months from intervention; it will be "late" when onset is had 6 months after intervention.

Object of the present invention are pharmaceutical compositions for epidural use in the therapeutic treatment of Failed Back Surgery Syndrome comprising sodium 2-mercaptoethane sulfonate and one or more carriers and/or diluents and/or excipients.

Epidural use of said compositions is meant for the treatment of lumbar pain in any form, e.g. chronic or acute and associated to any pathology, like e.g. failed back surgery syndrome, lumbosciatic pain or crural pain due to spinal disc herniation, tumoral lesions that compress the nerves, metabolic or post-radiotherapy radiculopathies.

The compositions of the present invention could be formulated with one or more carriers and/or diluents and/or excipients according to known techniques. Such carriers and/or diluents and/or excipients can be selected among those normally known in the state of the art and include: a) carriers, such as sodium citrate and calcium phosphate, b) diluents, water for injectable preparations, sterile saline solution c) excipients, such as sodium edetate.

The compositions will preferably be in the form of a solution or suspension.

The concentration of sodium 2-mercaptoethane sulfonate in the compositions is comprised, e.g., between 1 and 500 mg/ml, preferably between 50 and 100 mg/ml. The dosage of sodium 2-mercaptoethane sulfonate administered may vary depending on the patient's general conditions, the nature and seriousness of the pathology or disorder. Dosage should therefore take into account severity of the condition to be treated, and general physical conditions of the specific patient, as is well-known to those skilled in the art. Moreover, evidently said effective amount could, when required, be lowered or increased according to the responses of the treated patient.

Typically, the compositions for intraspinal use could contain an amount of sodium 2-mercaptoethane sulfonate comprised between 50 and 100 mg per dosage unit,

By the term "dosage unit" it is meant the amount of active principle per single epidural administration.

Hereinafter, experiments and examples are reported with the purpose of better illustrating what reported in the present description.

### EXAMPLES

### 1. Clinical study results

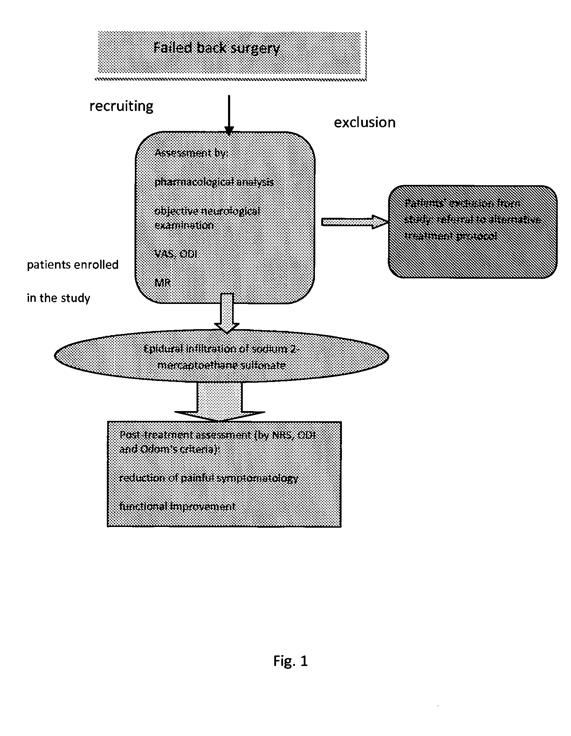

11 patients (7 males and 4 females), of ages ranging between 48 and 78 years, and suffering from FBSS, were studied (according to the operative diagram in Figure 1) for a 9-month period.

As criteria for inclusion in the treatment protocol were considered:
- *failed back surgery syndrome (FBSS)*
- *lumbar pain present for >3 months*
- *absence of improvement of pain with administration of oral pharmacological analgesics*/*opioids-based therapy;*
- *non-eligibility for lumbar revision surgery treatment.*

Exclusion criteria:
- *patients refusing treatment;*
- *carriers of infections localized at the level of the infiltration site;*
- *undergoing systemic infective processes;*
- *coagulation alterations.*

After having been evaluated under the clinical instrumental standpoint, the patient was subjected to infiltrative treatment upon coagulation testing and replacement of oral anticoagulant therapy (possibly taken by the patient), with low molecular weight heparin. Infiltration was conducted in Day Surgery regimen, inviting the patient to have it on an empty stomach. The injection was made above or below the level that had been the site of the surgical intervention, visible owing to the presence of a scar in the lumbar region and instrumentally displayed during outpatient care by MR imaging. The drug at issue, sodium 2-mercaptoethansulfonate, was contained in an ampoule with a 10% concentration; for each infiltration, 1 ml was collected and the concentration used was of 50 mg/ml in some cases and of 100 mg/ml in other cases. The entire infiltration procedure required 10-15 min.

Of the 11 patients, 2 had already undergone 3 surgical interventions to the lumbar rachis, other 2 had undergone 2 interventions, and 7 had undergone 1 intervention. In particular, 4 had undergone an herniectomy, 2 an hemilaminectomy, 2 an herniectomy plus an hemilaminectomy and 3 a vertebral stabilization intervention. The levels involved were comprised between L1 and S1, with an extension range of 2 to 5 levels.

In our sample being examined, three patients were carrying a medullary electrostimulator for antalgic purposes, and 1 patient was carrying an intratecal pump for opioids release; oft-times the presence of such devices is not associated to an improved control of painful symptomatology, and vice versa it was associated to a scarce response, with scarce NRS and ODI values.

Each patient was subjected, on average, to 2 (range comprised between 1 and 4) peridural infiltrations of sodium 2 mercaptoethane sulfonate (Figure 2).

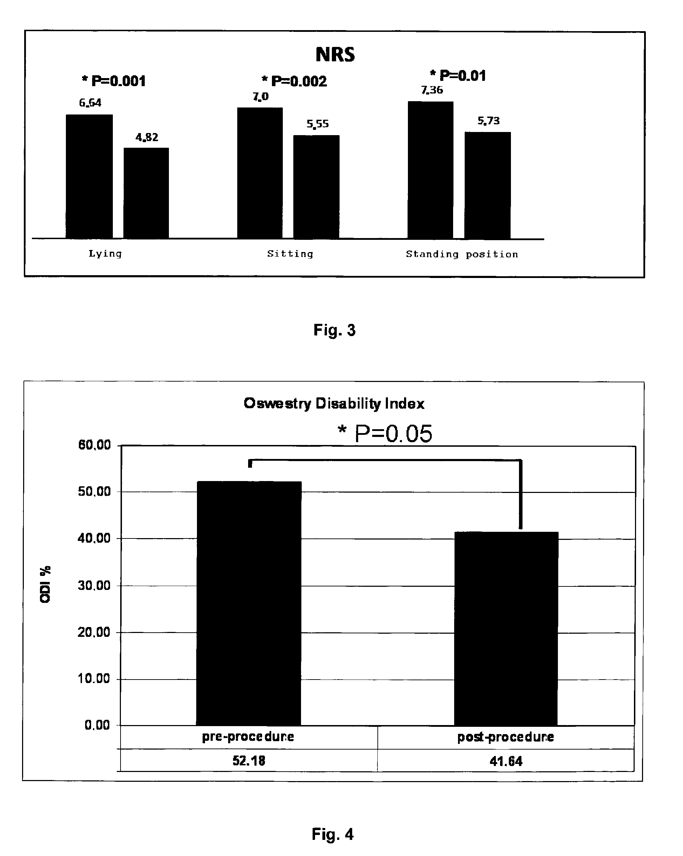

During an outpatient care visit, all patients were subjected to pain evaluation by visuo-analogic scales, via Numeric Rating Scales (NRS), considering its intensity in the sitting (Figure 2), standing and horizontal lying positions, and to assessment of lumbar rachis functionality (Oswestry Disability Index, ODI) before the procedure and at +1 week therefrom. The *Numeric Rating Scale (NRS)* was also studied. In particular, we asked to assign a value (comprised between 0 and 10) to pain intensity in a standing position, in horizontal posture, and while sitting; evaluation of the 3 parameters helped us in pain classification, and was aimed at determining musculoskeletal and gravity activation at different instants and the quantitative impact on pain intensity caused by the assuming of the different positions. During the outpatient care visit, the patient was invited to mark on the scale the number best representing the intensity of his/her pain, both before carrying out the infiltrative treatment and at +1 week from infiltration. Thus, it was possible to monitor variations in pain intensity following the treatment. Moreover, the patients filled the Oswestry Disability Index (ODI) questionnaire, which investigates on the influence that the painful symptomatology has in connection to the carrying out of daily activities (Figure 3). ODI represents one of the most specific measurements with regard to the monitoring of pathologies of the rachis. For the study, the Italian version 2.1 was used, comprised of nine questions (statements) related to: pain intensity, personal care, weight lifting ability, walking endurance, ability to keep a sitting position (time), ability to keep the standing position (time), sleep interruption due to pain, social life, travelling. Each question had six possible answers, for a maximum of 5 points per question. To the first answer a score equal to 0 was assigned, to the second one equal to 1, and so on until the sixth answer that would be equal to 5. the score is calculated as follows: TOTAL SCORE/(5x9) x 100.

Moreover, all patients were evaluated by Odom's criteria (Table 1), which on the basis of functional improvement define the result of the procedure carried out as: *Excellent* (Total relief of pre-procedure symptoms; relief of abnormal findings), Good (Minimal persistence of pre-procedure symptoms; abnormal findings unchanged or improved) *Fair* (Improvement of pre-procedure symptoms; other signs unchanged or slightly improved), or *Poor* (Signs and symptoms unchanged or exacerbated).

From data assessment, it emerged that pre-procedure pain (quantified with visuo-analogical scales NRS) was on average 7.0 in a sitting position, 7.36 standing and 6.64 in a horizontal position (Fig. 3). Following the procedure a significant decrease of mean values, respectively to 5.55 (P=0.002), 5.73 (P=0.01), and 4.82 (P=0.001) was observed. The disability index linked to pathologies of the lumbosacral rachis was measured by the Oswestry Disability Index (ODI), which on average went from 52.18% before the procedure to 41.64% (P=0.05) after the procedure (Fig. 4). The functional improvement, recorded by the patient answering the 9 questions posed, showed a score-improving trend when the infiltrative procedure was repeated more than once.

Out of 11 patients, Odom's criteria (Table 1) demonstrated "Good" results in 7 patients (63.6%), and "Fair" results in 4 patients (36.4%). No "Excellent" results were detected, but on the other hand in this category of patients they were not expected a *priori.* However, it should be stressed that there was no "Poor" result, i.e. no result with unchanged signs and symptoms.

**Table 1: Variation of Odom's criteria following the procedure: on 11 patients with FBSS, all perceived an improvement of the lumbar pain symptomatology; 7 of them with a minimal persistence of pre-procedure symptoms and 4 with symptom improvement. At the same time, after the procedure no patient exhibited unchanged or exacerbated symptoms.**

| ***Table 1: Odom's criteria in the population subject of study*** | | |
|---|---|---|
| **0** | **Excellent** | *Total relief of pre-procedure symptoms; relief of abnormal findings* |
| **7** | **Good** | *Minimal persistence of pre-procedure symptoms; abnormal findings unchanged or improved* |
| **4** | **Fair** | *Improvement of pre-procedure symptoms; other signs unchanged or slightly improved* |
| **0** | **Poor** | *Signs and symptoms unchanged or exacerbated* |

### REFERENCES

Denaro et al. Eur Spine J (2008) 17:1752-1756 Effectiveness of a mucolythic agent as a local adjuvant in revision lumbar spine surgery.
Borg et al. Pain (1994) 58:273-276 Hyaluronidase in the management of pain due to post-laminectomy scar tissue.

## Claims

1. Sodium 2-mercaptoethane sulfonate for epidural use in the treatment of lumbar pain.

2. Sodium 2-mercaptoethane sulfonate according to claim 1 for epidural use in the treatment of lumbar pain associated to Failed Back Surgery Syndrome.

3. Sodium 2-mercaptoethane sulfonate according to claim 1 for epidural use in the treatment of lumbar pain associated to a pathology selected from the group comprised of acute or chronic low back pain, lumbosciatic pain or crural pain due to spinal disc herniation, tumoral lesions, metabolic or post-radiotherapy radiculopathy.

4. A pharmaceutical composition comprising sodium 2-mercaptoethane sulfonate and one or more carriers and/or diluents and/or excipients, for epidural use in the treatment of lumbar pain.

5. The pharmaceutical composition for use according to the preceding claim for epidural use in the treatment of lumbar pain associated to Failed Back Surgery Syndrome.

6. The pharmaceutical composition for use according to claim 4 for epidural use in the treatment of lumbar pain associated to a pathology selected from the group comprised of acute or chronic low back pain, lumbosciatic pain o crural pain due to spinal disc herniation, tumoral lesions, metabolic or post-radiotherapy radiculopathy.

7. The pharmaceutical composition for use according to any one of the claims 4 to 6, in the form of a solution.

8. The pharmaceutical composition for use according to the preceding claim, wherein sodium 2-mercaptoethane sulfonate is in a concentration comprised between 1 and 500 mg/ml.

9. The pharmaceutical composition for use according to the preceding claim, wherein sodium 2-mercaptoethane sulfonate is in a concentration comprised between 50 and 100 mg/ml.

10. The pharmaceutical composition for use according to any one of the claims 4 to 9 wherein said carrier is selected from the group comprised of sodium citrate and calcium phosphate.

11. The pharmaceutical composition for use according to any one of the claims 4 to 9, wherein said diluent is a sterile saline solution or water for injectable preparations.

12. The pharmaceutical composition for use according to any one of the claims 4 to 9, wherein said excipient is sodium edetate.

13. The pharmaceutical composition for use according to any one of the claims 4 to 12 wherein sodium 2-mercaptoethane sulfonate is comprised between 50-100 mg per dosage unit.

14. The pharmaceutical composition for use according to any one of the claims 7 to 13, contained in a syringe for epidural injections.

## Patentansprüche

1. Natrium-2-Mercaptoethan-Sulfonat zur epiduralen Anwendung bei der Behandlung von Lumbalschmerzen.

2. Natrium-2-Mercaptoethan-Sulfonat gemäß Anspruch 1 zur epiduralen Anwendung bei der Behandlung von Lumbalschmerzen in Zusammenhang mit Postdiskotomiesyndrom.

3. Natrium-2-Mercaptoethan-Sulfonat gemäß Anspruch 1 zur epiduralen Anwendung bei der Behandlung von Lumbalschmerzen in Zusammenhang mit einer Symptomatik ausgewählt aus der Gruppe bestehend aus akutem oder chronischem Schmerz im unteren Rückenbereich, Lumboischialgie oder Schmerzen im Schenkel aufgrund von Bandscheibenvorfall, Tumorläsionen, metabolischer Radikulopathie oder Radikulopathie nach Strahlenbehandlung.

4. Arzneimittel umfassend Natrium-2-Mercaptoethan-Sulfonat und einen oder mehrere Träger und/oder Verdünnungsmittel und/oder Exzipienten zur epiduralen Anwendung bei der Behandlung von Lumbalschmerzen.

5. Arzneimittel zur Verwendung gemäß dem vorhergehenden Anspruch zur epiduralen Anwendung bei der Behandlung von Lumbalschmerzen in Zusammenhang mit Postdiskotomiesyndrom.

6. Arzneimittel zur Verwendung gemäß Anspruch 4 zur epiduralen Anwendung bei der Behandlung von Lumbalschmerzen in Zusammenhang mit einer Symptomatik ausgewählt aus der Gruppe bestehend aus akutem oder chronischem Schmerz im unteren Rückenbereich, Lumboischialgie oder Schmerzen im Schenkel aufgrund von Bandscheibenvorfall, Tumorläsionen, metabolischer Radikulopathie oder Radikulopathie nach Strahlenbehandlung.

7. Arzneimittel zur Verwendung gemäß einem der Ansprüche 4 bis 6 in Form einer Lösung.

8. Arzneimittel zur Verwendung gemäß dem vorhergehenden Anspruch, wobei Natrium-2-Mercaptoethan-Sulfonat in einer Konzentration zwischen 1 und 500 mg/ml vorliegt.

9. Arzneimittel zur Verwendung gemäß dem vorhergehenden Anspruch, wobei Natrium-2-Mercaptoethan-Sulfonat in einer Konzentration zwischen 50 und 100 mg/ml vorliegt.

10. Arzneimittel zur Verwendung gemäß einem der Ansprüche 4 bis 9, wobei der Träger ausgewählt ist aus der Gruppe bestehend aus Natriumcitrat und Calciumphosphat.

11. Arzneimittel zur Verwendung gemäß einem der Ansprüche 4 bis 9, wobei das Verdünnungsmittel eine sterile Kochsalzlösung oder Wasser für injizierbare Zubereitungen ist.

12. Arzneimittel zur Verwendung gemäß einem der Ansprüche 4 bis 9, wobei der Exzipient Natriumedetat ist.

13. Arzneimittel zur Verwendung gemäß einem der Ansprüche 4 bis 12, wobei Natrium-2-Mercaptoethan-Sulfonat zwischen 50 bis 100 mg pro Dosierungseinheit beträgt.

14. Arzneimittel zur Verwendung gemäß einem der Ansprüche 7 bis 13, enthalten in einer Spritze für epidurale Injektionen.

## Revendications

1. 2-Mercapto-éthane-sulfonate de sodium pour utilisation en épidurale dans le traitement d'une douleur lombaire.

2. 2-Mercapto-éthane-sulfonate de sodium, conforme à la revendication 1, pour utilisation en épidurale dans le traitement d'une douleur lombaire associée aux séquelles d'un échec chirurgical rachidien.

3. 2-Mercapto-éthane-sulfonate de sodium, conforme à la revendication 1, pour utilisation en épidurale dans le traitement d'une douleur lombaire associée à une pathologie choisie dans l'ensemble formé par les suivantes : douleur aiguë ou chronique du bas du dos, douleur lombosciatique ou douleur crurale due à une hernie discale, lésions tumorales, radiculopathie métabolique ou post-radique.

4. Composition pharmaceutique comprenant du 2-mercapto-éthane-sulfonate de sodium et un ou plusieurs véhicule(s) et/ou diluant(s) et/ou excipient(s), pour utilisation en épidurale dans le traitement d'une douleur lombaire.

5. Composition pharmaceutique pour utilisation conforme à la revendication précédente, pour utilisation en épidurale dans le traitement d'une douleur lombaire associée aux séquelles d'un échec chirurgical rachidien.

6. Composition pharmaceutique pour utilisation conforme à la revendication 4, pour utilisation en épidurale dans le traitement d'une douleur lombaire associée à une pathologie choisie dans l'ensemble formé par les suivantes : douleur aiguë ou chronique du bas du dos, douleur lombosciatique ou douleur crurale due à une hernie discale, lésions tumorales, radiculopathie métabolique ou post-radique.

7. Composition pharmaceutique pour utilisation conforme à l'une des revendications 4 à 6, sous la forme d'une solution.

8. Composition pharmaceutique pour utilisation conforme à la revendication précédente, dans laquelle le 2-mercapto-éthane-sulfonate de sodium se trouve en une concentration de 1 à 500 mg/mL.

9. Composition pharmaceutique pour utilisation conforme à la revendication précédente, dans laquelle le 2-mercapto-éthane-sulfonate de sodium se trouve en une concentration de 50 à 100 mg/mL.

10. Composition pharmaceutique pour utilisation conforme à l'une des revendications 4 à 9, dans laquelle ledit véhicule est choisi parmi du citrate de sodium et du phosphate de calcium.

11. Composition pharmaceutique pour utilisation conforme à l'une des revendications 4 à 9, dans laquelle ledit diluant est une solution salée stérile ou de l'eau pour préparations injectables.

12. Composition pharmaceutique pour utilisation conforme à l'une des revendications 4 à 9, dans laquelle ledit excipient est de l'édétate de sodium.

13. Composition pharmaceutique pour utilisation conforme à l'une des revendications 4 à 12, dans laquelle le 2-mercapto-éthane-sulfonate de sodium se trouve à raison de 50 à 100 mg par unité poso-logique.

14. Composition pharmaceutique pour utilisation conforme à l'une des revendications 7 à 13, contenue dans une seringue pour injections épidurales.
